# FASCICULE DE BREVET EUROPEEN

(11) **EP 1 487 371 B1**
(45) Date de publication et mention de la délivrance du brevet: **03.12.2008**
(21) Numéro de dépôt: 03722701.4
(22) Date de dépôt: 20.03.2003
(51) Int. Cl.: A61B 19/02

(54) **BOITIER D'EMBALLAGE ET DE PRESENTATION POUR UN ENSEMBLE D'OSTEOSYNTHESE, ENSEMBLE DE PROTECTION ET COFFRET DE PRESENTATION COMPRENANT UN TEL BOITIER.**
VERPACKUNGS- SOWIE DARSTELLUNGSGEHÄUSE FÜR OSTEOSYNTHESEIMPLANTAT, SCHUTZANORDNUNG UND PRÄSENTATIONSBEHÄLTER MIT SOLCH EINEM GEHÄUSE
PACKAGING AND DISPLAY BOX FOR AN OSTEOSYNTHESIS ASSEMBLY, PROTECTIVE SET AND DISPLAY CASE COMPRISING SAME

(30) Priorité: 22.03.2002 FR 0203594
(43) Date de publication de la demande: 22.12.2004
(73) Titulaire: Caron, Philippe, Papeete 98713, BP 1040 Tahiti (FR)
(72) Inventeur: Caron, Philippe, Papeete 98713, BP 1040 Tahiti (FR)
(74) Mandataire: Cardy, Sophie Marie
(86) Numéro de dépôt international: PCT/FR2003/000885
(87) Numéro de publication internationale: WO 2003/079918

(56) Documents cités:
- EP-A- 0 290 138
- WO-A-00/57810
- WO-A-01/52762
- DE-U- 29 705 944
- US-A- 5 732 821

## Description

La présente invention concerne un boîtier d'emballage et de présentation pour un ensemble d'ostéosynthèse, ledit ensemble comprenant au moins une plaque et au moins une vis. Le document EP-A-0290138 divulgue un boîtier selon le préambule de la revendication 1.

Les vis et les plaques d'ostéosynthèse permettent d'effectuer une réduction de fracture osseuse, à savoir de remettre et de maintenir en position deux fragments osseux l'un par rapport à l'autre.

De telles vis et plaques d'ostéosynthèse sont notamment classiquement notamment utilisées au cours d'interventions de chirurgie maxillo-faciale, que ce soit à visée réparatrice pour réduire une fracture accidentelle, ou à visée fonctionnelle et/ou esthétique comme dans le cas des ostéotomies de la mandibule et du maxillaire.

Au cours de ces interventions chirurgicales, le chirurgien dispose d'un lot de plaques et d'un lot de vis de fixation qui ont été au préalable sorties de leur emballage respectif (par exemple d'un récipient contenant les plaques et d'un autre récipient contenant les vis de fixation) avant d'être stérilisées puis placées sur un plateau qui reste accessible tout au long de l'intervention.

Selon le type d'intervention à effectuer, le lot de plaques peut comprendre de une à plusieurs plaques, identiques ou non, et le lot de vis peut comprendre de une à plusieurs vis, identiques ou non. Ces lots seront généralement constitués de plus d'une pièce car même dans le cas où une seule pièce est requise pour l'intervention, il est nécessaire de prévoir au moins une autre pièce de rechange au cas où la première pièce manipulée a été abîmée mécaniquement ou bien si son état d'asepsie n'est plus satisfaisant.

En outre, même dans le cas ou le type de plaques et le type de vis à utiliser est préétabli, il peut rester des incertitudes quant aux dimensions les plus adaptées à la morphologie du patient, notamment en ce qui concerne la longueur des vis à utiliser.

En conséquence, de nombreuses manipulations sont nécessaires avant, pendant et après l'intervention chirurgicale en relation avec ces éléments (plaques et vis) formant l'ensemble d'ostéosynthèse :
- avant l'intervention :le personnel de bloc doit préparer les éléments de l'ensemble d'ostéosynthèse, c'est-à-dire connaître le ou les types de vis et de plaques à prévoir, ainsi que leur nombre, les chercher dans le stock, puis ces éléments sont stérilisés et mis dans une enveloppe étanche ; ces éléments restent ensuite en attente jusque peu de temps avant l'intervention : à ce moment là, on ouvre l'enveloppe étanche et les éléments sont alors placés sur le plateau précité ;
- pendant l'intervention :tout ou partie des éléments de l'ensemble d'ostéosynthèse vont être saisis par une pince ou un autre instrument adapté avant d'être mis en place et fixés sur le patient grâce aux instruments prévus à cet effet ; l'aller-retour de cette pince (ou de cet autre instrument) entre le patient, ou les instruments précités, et le plateau engendre des risques de souillure et de contamination des éléments non encore utilisés restés sur le plateau ; et
- après l'intervention : du fait de ce qui précède, les éléments restants qui ne sont pas détériorés du point de vue mécanique vont devoir de nouveau subir une étape de nettoyage et de stérilisation, qu'ils aient été touchés par un instrument ou non, avant de retourner dans le stock dans l'attente d'être sélectionnés en vue d'une prochaine intervention ; en effet, ces éléments restant ne sont de tout manière plus stériles.

La présente invention a pour objectif de résoudre les problèmes précités afin de permettre une diminution des manipulations à réaliser au bloc opératoire, y compris en relation avec la stérilisation, de façon à fournir au chirurgien un ensemble d'ostéosynthèse directement utilisable au bloc opératoire, tout en garantissant la qualité et la stérilité du matériel formant l'ensemble d'ostéosynthèse.

A cet effet, selon l'invention, on propose un boîtier d'emballage et de présentation défini dans le jeu de revendications annexé et qui comporte une partie de fond et au moins une partie de couvercle, ladite partie de fond comprenant une paroi de fond et une paroi latérale prolongeant vers le haut la paroi de fond sur toute sa périphérie en délimitant un espace de rangement, ladite partie de couvercle étant rendue mobile par rapport à ladite partie de fond, entre une position d'ouverture et une position de fermeture, par des moyens fonctionnels, ledit boîtier comportant en outre des moyens de fermeture réversible disposés sur ladite partie de fond et sur ladite partie de couvercle et qui sont aptes à coopérer dans ladite position de fermeture, et ledit boîtier comportant en outre des moyens de logement dudit ensemble d'ostéosynthèse, lesdits moyens de logement étant aptes à être retenus dans ledit espace de rangement et comportant au moins un insert, lesdits moyens de logement étant munis d'au moins une cavité destinée à recevoir au moins une plaque d'ostéosynthèse et d'au moins un alvéole destiné à recevoir au moins une vis d'ostéosynthèse.

On comprend que le boîtier selon l'invention permet ainsi de fournir un ensemble d'ostéosynthèse déjà préparé et assorti, directement utilisable au bloc opératoire.

Un tel boîtier, réalisé classiquement en matière plastique notamment pour des raisons de coûts et de légèreté, doit répondre aux critères d'asepsie du bloc opératoire. En effet, dans le cadre de son utilisation au cours d'opérations chirurgicales, le boîtier est directement amené en salle d'opération aseptique.

A cet effet, selon la présente invention, il est prévu que ledit boîtier selon l'invention comprend en outre un film thermoscellable refermant ledit espace de rangement. De cette manière, avec une opération de stérilisation effectuée sous rayonnement, de préférence par rayonnement aux rayons gamma, on n'altère pas le film de matière plastique thermoscellable et on garantit la conservation de l'état de stérilité de l'espace de rangement qui reste obturé de manière étanche par le film thermoscellable.

Selon une solution préférentielle permettant la traçabilité du boîtier et des éléments qu'il contient et qui composent l'ensemble d'ostéosynthèse, il est prévu que la face de la paroi de fond tournée en direction opposée audit espace de rangement est munie d'un support d'identification portant des informations en relation avec ledit ensemble d'ostéosynthèse, en particulier sous la forme d'un code à barres. Ce support d'identification est par exemple constitué d'une étiquette en une ou plusieurs parties.

La présente invention porte également sur un ensemble de protection intégrant le boîtier précité, ce dernier étant logé dans une barquette délimitant un logement apte à recevoir ledit boîtier d'emballage, ladite barquette étant munie d'un film thermoscellable refermant ledit logement.

Selon un autre aspect de la présente invention, ledit ensemble de protection, tel que défini dans le paragraphe qui précède, comprend en outre un étui de protection et de conditionnement apte à entourer ladite barquette.

De cette manière, on multiplie les couches protégeant mécaniquement et/ou de manière aseptique le boîtier et son contenu, à savoir ledit ensemble d'ostéosynthèse.

Selon un mode de réalisation préférentiel, ledit étui de protection porte des moyens de reconnaissance visuelle du type d'ensemble d'ostéosynthèse qui est contenu dans ledit boîtier d'emballage. De cette manière, il est possible de savoir, rien qu'en regardant ledit étui de protection quel est la composition et/ou l'indication chirurgicale de l'ensemble d'ostéosynthèse contenu dans le boîtier.

De préférence, ledit étui de protection comporte une partie de fond et une partie de couvercle mobiles l'un par rapport à l'autre entre une position d'ouverture et une position de fermeture, et ledit étui de protection comporte en outre des moyens de fermeture réversible disposés sur ladite partie de fond et sur ladite partie de couvercle qui sont aptes à coopérer dans ladite position de fermeture.

La présente invention porte également sur un coffret de présentation pour la distribution d'ensembles d'ostéosynthèse comprenant une partie de fond équipée de compartiments, au moins un compartiment comprenant un ensemble de protection tel qu'il a été défini précédemment, et au moins une partie de couvercle mobile par rapport à ladite partie de fond et destinée à refermer de manière réversible au moins partiellement l'espace délimité par la partie de fond.

L'invention sera mieux comprise, et les caractéristiques secondaires et leurs avantages apparaîtront au cours de la description d'un mode de réalisation donnée ci-dessous à titre d'exemple.

Il est entendu que la description et les dessins ne sont donnés qu'à titre indicatif et non limitatif.

Il sera fait référence aux dessins annexés, dans lesquels :
- la figure 1 est une vue en perspective depuis un des coins avant et depuis le dessus d'un boîtier d'emballage et de présentation pour un ensemble d'ostéosynthèse conforme à la présente invention, dans une position intermédiaire entre la position de fermeture et la position d'ouverture,
- la figure 2 est une vue en projection depuis le dessus de la partie de fond du boîtier de la figure 1,
- la figure 3 est une vue en coupe selon la direction III-III de la figure 2,
- la figure 4 est une vue en coupe selon la direction IV-IV de la figure 2,
- la figure 5 est une vue schématique partielle, en perspective, d'une alternative de réalisation de la partie de fond du boîtier d'emballage selon la présente invention,
- la figure 6 est une vue en perspective depuis l'arrière du boîtier de la figure 1, dans sa position d'ouverture,
- la figure 7 est une vue en perspective depuis l'un des coins et depuis le dessus d'une barquette contenant le boîtier selon l'invention afin de former un ensemble de protection,
- la figure 8 est une vue en perspective depuis l'un des coins et depuis le dessus d'un étui de protection contenant la barquette de la figure 7, dans sa position d'ouverture, afin de former un ensemble de protection, et
- la figure 9 est une vue en perspective depuis l'un des coins et depuis le dessus d'un coffret de présentation ouvert dans lequel sont disposé des ensembles de protection conformes à la figure 8.

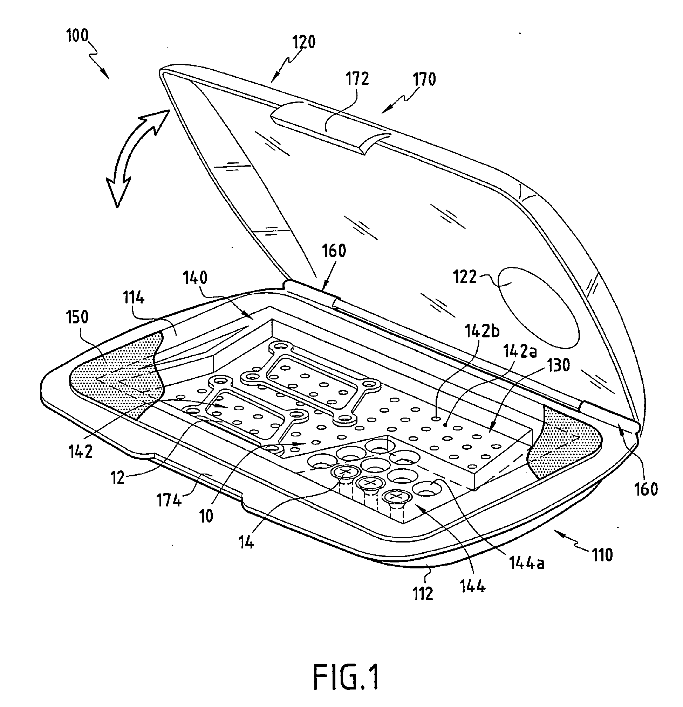

Le boîtier d'emballage et de présentation 100 représenté sur la figure 1 comporte une partie de fond 110 et une partie de couvercle 120.

La partie de fond 110 et la partie de couvercle 120 présentent toutes les deux une forme généralement rectangulaire avec une même largeur et une même longueur de façon à ce que la partie de couvercle 120 soit susceptible de venir refermer l'espace 130 délimité dans la partie de fond 110.

Il est entendu que d'autres formes sont également envisageables pour la partie de fond 110 et pour la partie de couvercle 120 sans pour autant sortir du cadre de la présente invention, des formes circulaires, ovales, oblongues, carrées, triangulaires, en forme de parallélogramme ou de polygone ... peuvent être utilisées de la même manière.

La partie de fond 110 est constituée d'une paroi de fond 112 prolongé vers le haut le long de toute sa périphérie par une paroi latérale 114 continue se refermant sur elle-même en ceinturant l'espace 130. Ainsi, la paroi de fond 112 et la paroi latérale 114 délimitent l'espace de rangement 130 destiné à recevoir un ensemble d'ostéosynthèse 10.

Sur la figure 1, l'ensemble d'ostéosynthèse 10 comporte deux plaques d'ostéosynthèse 12, formées chacune par une plaque plate en forme de losange, et trois vis 14.

Afin de présenter l'ensemble d'ostéosynthèse 10 d'une manière qui permette à la fois de bien repérer séparément chacun des éléments de l'ensemble d'ostéosynthèse 10 et de pouvoir les saisir individuellement aisément, l'espace de rangement 130 contient des moyens de logement sous la forme d'un insert 140 susceptible d'être réalisé en un plastique rigide moulé, tout comme la partie de fond 110 et la partie de couvercle 120.

L'insert 140 présente une forme extérieure complémentaire de celle de l'espace de rangement 130 délimité par la partie de fond 110 de sorte qu'en usage normal, l'insert 140 reste logé dans l'espace de rangement 130.

De manière plus précise, comme il apparaît sur la figure 1, l'insert 140 comporte deux zones distinctes : une première zone délimitant une cavité 142, dans laquelle sont placées les deux plaques 12 visibles sur la figure 1, et une deuxième zone 144 comportant plusieurs alvéoles 144a pour le logement individuel des vis 14.

Sur la figure 1, la cavité 142 comporte un fond formé d'une face de support pour les plaques d'ostéosynthèse, cette face de support 142a étant munie de zones en creux 142b sous la forme de petites dépressions.

Ces zones en creux 142b sont susceptibles d'être utilisées comme l'un des éléments de moyens de retenue destinés à coopérer de manière réversible avec la plaque d'ostéosynthèse 12 pour retenir cette dernière sur la face de support 142a.

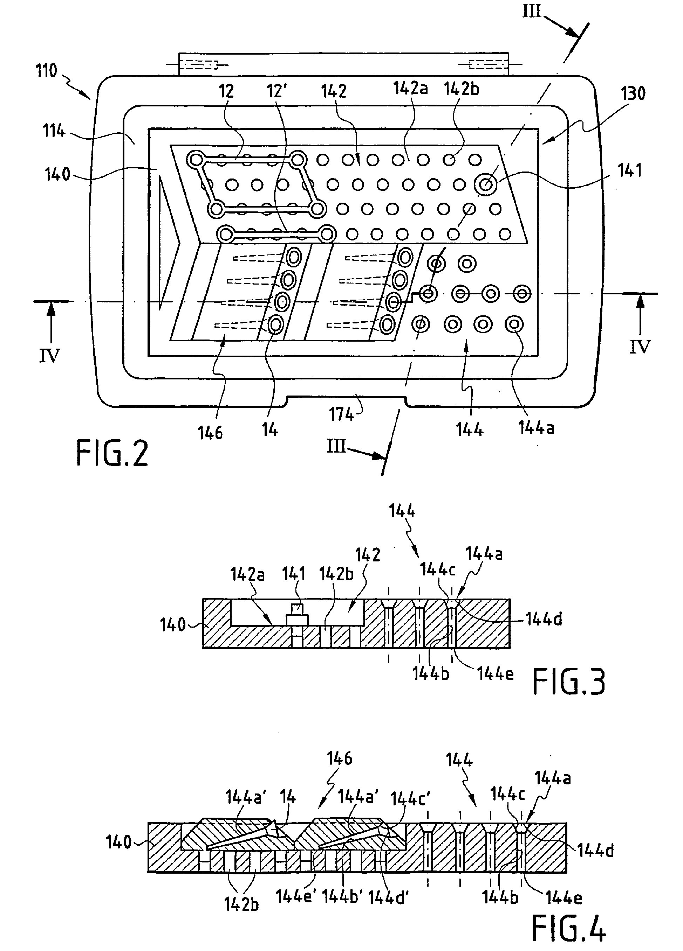

Ces moyens de retenue sont plus particulièrement représentés sur les figures 2 et 3 : des picots 141 comportant deux portions de tiges sont susceptibles de venir se loger dans une de ces dépressions 142b, la portion de tige faisant saillie hors de la zone en creux 142b pouvant être reçue dans l'un des alésages d'une plaque (plaque 12 en forme de losange et plaque 12' linéaire sur la figure 2). Ces picots 141 forment l'autre des éléments des moyens de retenue précités puisque chacun d'entre eux permet, en combinaison avec l'une des dépressions 142b, de maintenir une plaque contre la face de support 142a, à un emplacement bien déterminé.

A titre d'alternative de réalisation, la face de support 142a de la cavité 142 de l'insert 140 peut également être munie à la fois de zones en creux et de zones en saillie.

Quelle qu'en soit sa forme, ce relief permet de surélever légèrement les plaques afin de faciliter leur préhension à l'aide d'un instrument tel qu'une pince, et notamment une pince spécialement adaptée à la préhension de ce type de plaques.

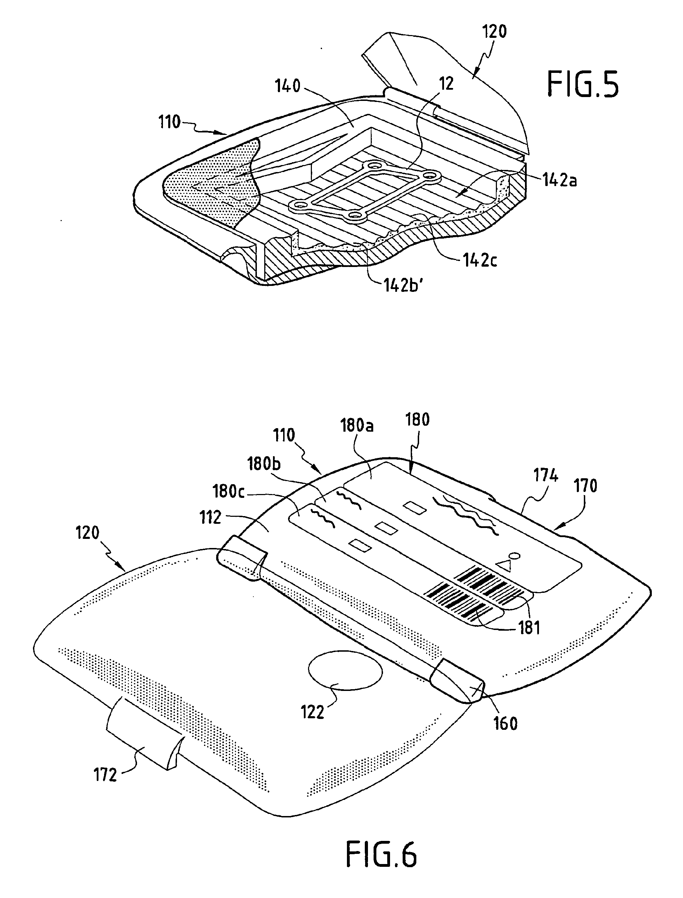

En particulier, selon une autre alternative de réalisation préférentielle, la face de support 142a de la cavité 142 est munie de nervures 142c et de rainures 142b' parallèles entre elles, de façon à former une surface cannelée sur laquelle repose la plaque d'ostéosynthèse 12, comme on peut le voir sur la figure 5.

D'autres reliefs présentant des creux et des zones en saillie peuvent bien entendu être utilisés de la même manière.

Si l'on se réfère à nouveau à la figure 1, la deuxième zone 144 de l'insert 140, qui est illustrée comme formant un des angles de l'espace de rangement 130, comporte dix alvéoles 144a formés d'une empreinte en creux présentant une forme complémentaire de la vis destinée à être reçue dans cet alvéole.

Ces alvéole 144a sont réalisés selon une empreinte présentant une forme permettant à la tête de la vis 14 d'être située dans l'ouverture de cette empreinte 144a. Plus précisément, la tête de la vis 14 est au niveau, légèrement au dessus, ou légèrement en dessous du plan de l'ouverture 144d de l'empreinte 144a, afin que l'on puisse en tout état de cause accéder à la tête de la vis 14 depuis cette ouverture 144d.

En particulier, comme on peut le voir sur les figures 3 et 4, les alvéoles 144a sont formés d'une portion cylindrique de section circulaire 144b et, au niveau de l'ouverture de l'alvéole 144a, d'une portion en forme de tronc de cône 144c, le tronc de cône allant en s'élargissant depuis ladite portion cylindrique 144b en direction de l'ouverture 144d de l'alvéole 144a.

Dans le cas des alvéoles 144a présentés sur la figure 1, ces alvéoles 144a sont dirigés selon un axe vertical perpendiculaire à la face de support 142a.

C'est aussi le cas des alvéoles 144a représentés sur les figures 2 à 4 et qui sont situés dans la deuxième zone 144 délimitée dans l'angle situé à droite et en bas de la figure 2 de l'espace de rangement 130.

Cette forme d'alvéole ne peut pas être utilisée lorsque les vis présentent une longueur supérieure à l'épaisseur de l'insert 140.

Dans ce cas, on utilise une troisième zone 146 de l'insert 140 qui est visible sur les figures 2 et 4. dans ce cas, les alvéoles 144a' sont orientés selon une direction longitudinale s'étendant depuis l'ouverture 144d' jusqu'au fond 144e', cette direction longitudinale étant inclinée par rapport à un plan parallèle à la paroi de fond 112 ou à la surface de support 142a.

De cette manière, la distance séparant le fond 144e' de l'ouverture 144d' de cet alvéole incliné 144a' formé d'une empreinte en creux est plus grande que la hauteur de l'insert 140 et que la hauteur de la paroi latérale 114 de la partie de fond 112.

Il est à noter qu'au lieu d'avoir une portion cylindrique 144b de section circulaire, cette portion peut également présenter une forme de tronc de cône allant en s'amincissant en direction du fond 144e : c'est le cas illustré sur les figures 2 et 4 pour les alvéoles inclinés 144a'. Dans ce cas, la portion 144b' destinée à recevoir la tige de la vis 14 est beaucoup moins évasée que la portion tronconique 144c' destinée à recevoir la tête de la vis.

Dans le cas du mode de réalisation illustré à la figure 1, les moyens de logement, permettant le rangement de l'ensemble d'ostéosynthèse 10, sont constitués de l'insert 140 qui est formé en une seule pièce, laquelle présente à la fois la première zone 142 et à la fois la deuxième zone 144.

Toutefois, l'espace de rangement 130 peut contenir plusieurs inserts formant lesdits moyens de logement, en l'occurrence au moins un premier insert équipé de la cavité 142 formant la première zone et au moins un deuxième insert équipé dudit alvéole 144a ou de plusieurs alvéoles 144a afin de former la deuxième zone 144.

Dans le cas du mode de réalisation illustré sur les figures 2 à 4, outre un premier insert formant la première zone 142 et un deuxième insert formant la deuxième zone 144, les moyens de logement comprennent en outre un troisième insert 146 formant la troisième zone précitée et délimitant au moins un alvéole 144a' destiné à recevoir une vis d'ostéosynthèse de plus grande longueur que les alvéoles 144a du deuxième insert 144 (deuxième zone).

Il est entendu qu'un tel troisième insert 146 peut être soit formé d'une seule pièce avec l'insert 140, soit être formé d'une ou plusieurs pièces séparées.

Selon une variante de réalisation non illustrée, les alvéoles 144a' de la troisième zone 146 de l'insert 140, qui sont destinés à recevoir des vis longues peuvent également être constitués d'un logement formé d'une surface en creux munie de deux pans inclinés entre eux et par rapport à la face de support 142a, la tige et la tête d'une vis d'ostéosynthèse étant respectivement destinées à venir prendre appui sur l'un ou l'autre des deux pans inclinés.

Ainsi, on comprend que, sans modifier la partie de fond 110, il est possible de loger un ou plusieurs inserts dévolus au logement d'une ou plusieurs plaques d'ostéosynthèse et d'une ou plusieurs vis d'ostéosynthèse, ces plaques et ces vis pouvant être différentes ou identiques entre elles.

Il est en outre prévu qu'un film thermo-scellable 150 recouvre le bord libre de la paroi latérale 114 de la partie de fond 110, afin d'en refermer l'espace de rangement 130.

La présence de ce film permet de garantir la stérilité de l'espace de rangement 130 des éléments qu'il contient tout en garantissant visuellement que cet espace de rangement 130 n'a pas déjà été ouvert au préalable.

La partie de couvercle 120 (visible sur la figure 1) est formée principalement d'une paroi destinée à recouvrir l'ouverture de l'espace de rangement 130.

Afin de pouvoir visualiser l'intérieur du boîtier d'emballage 100, il est prévu que la partie de couvercle 120 est au moins partiellement transparente, de préférence sur toute la surface en regard de l'ouverture de la partie de fond 110, c'est-à-dire en regard de tout l'espace de rangement 130.

De façon avantageuse, il est prévu en outre que la partie de couvercle 120 est équipée d'une loupe 122 intégrée dans sa paroi.

Cette loupe 122 permet, avant l'ouverture du boîtier 100, de visualiser de manière grossie la zone de l'espace de rangement 130 située en regard de cette loupe 122.

De manière originale, il est également possible d'utiliser cette loupe 122 pour l'agrandissement d'une image ou d'une inscription portée par une pastille qui est disposée sur la face interne de la partie de couvercle 120, en regard de cette loupe 122 (cas de figure non illustré).

La partie de couvercle 120 est rendue mobile par rapport à la partie de fond 110 au niveau d'une charnière 160 qui est illustrée de manière à former un pivot

Il est entendu que d'autres types d'éléments fonctionnels aptes à rendre mobile la partie de couvercle 120 par rapport à la partie de fond 110 pourraient être utilisés de la même manière que cette charnière 160 : par exemple, on peut utiliser un ensemble à glissière, rendant la partie de couvercle coulissante par rapport à la partie de fond, ou encore un pivot présentant un axe non pas parallèle au bord longitudinal du rectangle constituant la forme générale de la partie de couvercle 120 et de la partie de fond 110, mais un pivot présentant un axe perpendiculaire à la face de support 142a ou à la paroi de fond 112.

Afin de maintenir le boîtier d'emballage de présentation 100 selon la présente invention en position de fermeture (non illustrée), il est également prévu que la partie de fond 110 et la partie de couvercle 120 présentent des moyens de fermeture 170.

Dans le cas des modes de réalisation illustrés, ces moyens de fermeture 170 sont composés (voir figures 1 et 6) d'un système par clipsage ou par complémentarité de forme. Plus précisément, les moyens de fermeture 170 comportent une languette 172 disposée le long du bord de la partie de couvercle 120 qui est opposé à la charnière 160, cette languette 172 pouvant coopérer par complémentarité de forme avec une échancrure 174 disposée le long du bord de la paroi latérale 114 de la partie de fond 110, ce bord étant tourné en direction opposée de la paroi de fond 112 et de la charnière 160.

Selon un autre aspect de la présente invention, comme il apparaît sur la figure 6, la face de la paroi de fond 112 tournée en direction opposée audit espace de rangement 130 est munie d'une étiquette 180.

Cette étiquette 180 forme un support d'identification portant des informations en relation avec ledit ensemble d'ostéosynthèse 10, en particulier sous la forme d'un code à barres.

Plus précisément, l'étiquette 180 comporte trois portions 180a, 180b et 180c, chacune d'ente elles portant la référence de la société qui aura préparé et stérilisé l'ensemble d'ostéosynthèse 10 situé dans le boîtier 100.

Ces portions 180a, 180b et 180c comportent toute une série d'informations parmi lesquelles le numéro de série du boîtier 100, le numéro de lot et la référence de chacun des éléments (plaques 12 et vis 14) composant l'ensemble d'ostéosynthèse 10, des informations en relation avec la position de chacun des éléments de l'ensemble d'ostéosynthèse parmi les différents logements (cavité 142 et alvéoles 144a, 144a').

Ainsi, après l'utilisation de certains des éléments composant l'ensemble d'ostéosynthèse 10 qui est logé dans le boîtier 100, il est alors possible de connaître les éléments manquants pour réapprovisionner la boîte 100.

En outre, les portions 180a, 180b et 180c de l'étiquette 180 portant les mêmes informations, la portion 180a va être gardée pour archivage, tandis que la portion 180b sera collée sur le dossier du patient et la portion 180c pourra être utilisée pour le compte rendu de l'opération.

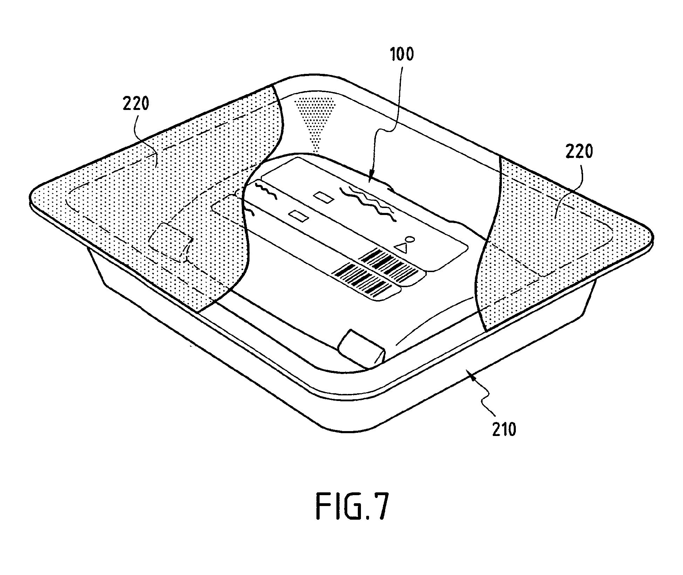

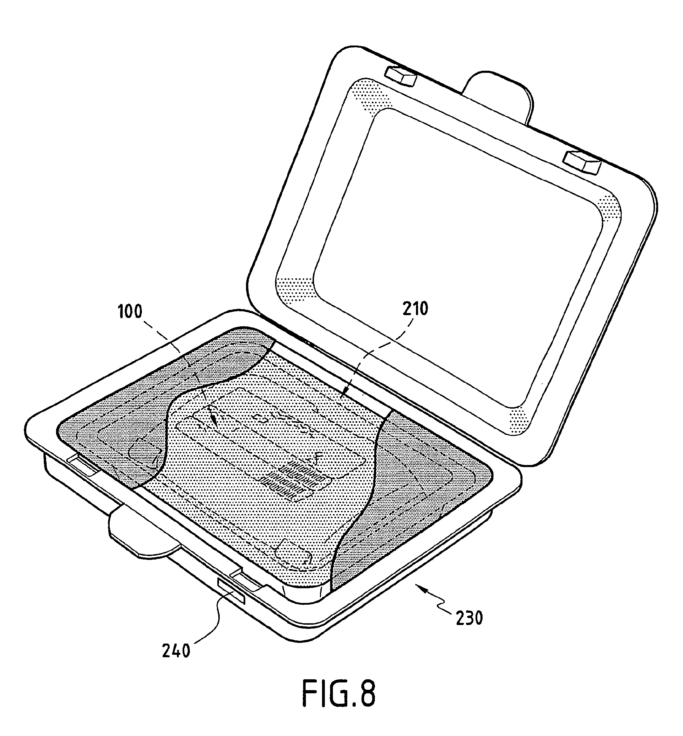

Afin de protéger le boîtier d'emballage de la présentation 100 durant son transport et avant l'utilisation de l'ensemble d'ostéosynthèse 10 qu'il comporte, la présente invention porte également sur un ensemble de protection 200 qui comporte, outre le boîtier d'emballage 100 tel que décrit précédemment, également :
- une barquette 210 (voir figure 7) réalisée de préférence en matière plastique transparente ou translucide, cette barquette 210 délimitant un logement apte à recevoir le boîtier 100 comme il apparaît sur la figure 7. Cette barquette 210 de forme générale rectangulaire se compose également d'une paroi de fond et d'une paroi latérale continue et prolongeant vers le haut la bordure de la paroi de fond, un film thermo-scellable 220 venant refermer de manière étanche l'espace délimité par cette barquette 210. De préférence, le boîtier d'emballage 100 est disposé dans l'espace délimité par la barquette 210, l'étiquette 180 étant tournée en direction du film thermo-scellable 220 afin que cette étiquette 180 au travers du film thermoscellable reste visible.
- un étui de protection et de conditionnement 230 (voir figure 8) réalisé également en matière plastique de préférence transparente ou translucide, cet étui 230 étant également formé d'une partie de fond et d'une partie de couvercle, mobiles entre elles entre une position d'ouverture et une position de fermeture, une étiquette extérieure 240 située de préférence sur la tranche de l'étui 230 permettant de former des moyens de reconnaissance visuelle du type d'ensemble d'ostéosynthèse 10 qui est contenu dans le boîtier d'emballage 100 logé dans la barquette 210, elle-même placée dans la partie de fond de l'étui 230. A cet effet, un code couleur porté par cette étiquette extérieure 240 pourra désigner un type particulier d'ensemble d'ostéosynthèse 10.

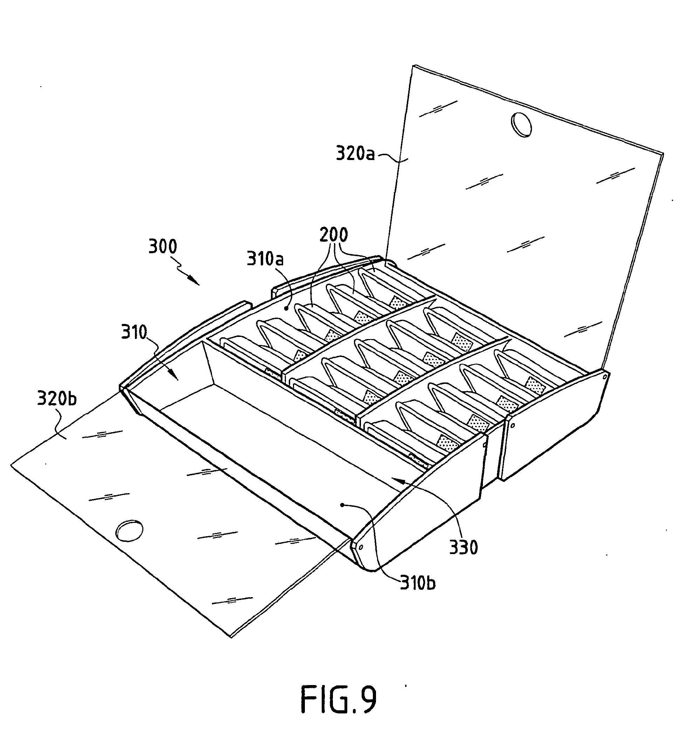

Pour faciliter la distribution des ensembles de protection 200 comprenant chacun un boîtier d'emballage de présentation 100, la présente invention propose également l'utilisation d'un coffret 300 (voir figure 9) comportant plusieurs compartiments 310a destinés à recevoir des ensembles de protection 200 et d'un compartiment 310b pouvant recevoir par exemple des instruments chirurgicaux devant être utilisés au cours de l'intervention utilisant l'ensemble d'ostéosynthèse 10 contenu dans le boîtier 100 de l'un des ensembles de protection 200.

Ainsi, grâce à l'étiquette 240 située sur chacun ces étuis de protection et de conditionnement 230 formant l'enveloppe extérieure des ensembles de protection 200, il est possible de connaître rapidement et simplement le type particulier d'ensemble d'ostéosynthèse 10 contenu dans chaque ensemble de protection 200, afin de choisir dans le coffret 300 l'étui contenant le boîtier d'emballage de présentation 100 qui aura été pourvu de l'ensemble d'ostéosynthèse qui est recherché.

Sur la figure 9, le coffret 300 présente également deux parties de couvercle 320a et 320b qui sont toutes les deux montées pivotantes par rapport au bord libre de la paroi latérale de la partie de fond 310 afin de refermer de manière réversible l'espace 330 délimité par la partie de fond 310.

Il est à noter que les éléments formant l'ensemble d'ostéosynthèse montré en relation avec le mode de réalisation décrit précédemment, ne doivent pas se limiter à du matériel pour réaliser une ostéotomie, notamment de la mandibule, mais qu'il convient de considérer également d'autres interventions de chirurgie maxillo-faciale, ou plus généralement toute intervention chirurgicale d'ostéosynthèse.

Ainsi, de manière plus générale, l'ensemble d'ostéosynthèse peut comprendre une (des) plaque(s) et/ou une (des) vis et/ou une (des) broches et/ou un (des) fil(s) métallique(s).

## Revendications

1. Boîtier d'emballage et de présentation (100) pour un ensemble d'ostéosynthèse (10), ledit ensemble d'ostéosynthèse (10) comprenant au moins une plaque (12) et au moins une vis d'ostéosynthèse (14), comportant une partie de fond (110) et au moins une partie de couvercle (120), ladite partie de fond comprenant une paroi de fond (112) et une paroi latérale (114) prolongeant vers le haut la paroi de fond (112) sur toute sa périphérie en délimitant un espace de rangement (130), ledit boîtier comportant en outre un film thermoscellable (150) refermant ledit espace de rangement (130) et des moyens de logement (140) dudit ensemble d'ostéosynthèse (10), **caractérisé en ce que** ladite partie de couvercle (120) est rendue mobile par rapport à ladite partie de fond, entre une position d'ouverture et une position de fermeture, par des moyens fonctionnels (160), **en ce que** ledit boîtier comporte en outre des moyens de fermeture (170) réversible disposés sur ladite partie de fond (110) et sur ladite partie de couvercle (120) et qui sont aptes à coopérer dans ladite position de fermeture, ,et **en ce que** lesdits moyens de logement (140) sont aptes à être retenus dans ledit space de rangement (130) et comportent au moins un insert (140, 144, 146), lesdits moyens de logement étant munis d'au moins une cavité (142) destinée à recevoir au moins une plaque d'ostéosynthèse (12) et d'au moins un alvéole (144a, 144a') destiné à recevoir au moins une vis d'ostéosynthèse (12).

2. Boîtier (100) selon la revendication 1, **caractérisé en ce que** ladite cavité (142) comporte une face de support (142a) de ladite plaque d'ostéosynthèse (12) qui est munie de zones en creux (142b, 142b').

3. Boîtier (100) selon la revendication 2, **caractérisé en ce que** ladite face de support (142a) est en outre munie de zones en saillie (142c).

4. Boîtier (100) selon la revendication 3, **caractérisé en ce que** ladite face de support de ladite plaque d'ostéosynthèse est munie de nervures (142c) et de rainures (142b').

5. Boîtier (100) selon l'une quelconque des revendications précédentes, **caractérisé en ce que** ladite cavité (142) comporte une face de support (142a) de ladite plaque d'ostéosynthèse (12) qui est munie de moyens de retenue (142b, 141) destinés à coopérer de manière réversible avec ladite plaque d'ostéosynthèse (12).

6. Boîtier (100) selon l'une quelconque des revendications précédentes, **caractérisé en ce que** la face de la paroi de fond (112) tournée en direction opposée audit espace de rangement (130) est munie d'un support d'identification (180) portant des informations en relation avec ledit ensemble d'ostéosynthèse (10), en particulier sous la forme d'un code à barres (181).

7. Boîtier (100) selon l'une quelconque des revendications précédentes, **caractérisé en ce** lesdits moyens de logement sont formés d'un insert (140) en une seule pièce.

8. Boîtier (100) selon l'une quelconque des revendications 1 à 6, **caractérisé en ce** lesdits moyens de logement comportent au moins un premier insert (140) équipé de ladite cavité (142) et au moins un deuxième insert (144) équipé dudit alvéole (144a).

9. Boîtier (100) selon la revendication 8, **caractérisé en ce que** lesdits moyens de logement comportent en outre un troisième insert (146) délimitant au moins un alvéole (144a') destiné à recevoir une vis d'ostéosynthèse (14).

10. Boîtier (100) selon l'une quelconque des revendications précédentes, **caractérisé en ce que** ledit alvéole (144a, 144a') est formé d'une empreinte en creux présentant une forme complémentaire de ladite vis (14) de sorte que la tête de la vis (14) est située dans l'ouverture (144d) de ladite empreinte.

11. Boîtier (100) selon la revendication 10, **caractérisé en ce que** ladite empreinte en creux (144a') présente une direction longitudinale depuis son ouverture (144d') jusqu'à son fond (144e'), ladite direction longitudinale étant inclinée par rapport à ladite paroi de fond (112) de sorte que la distance séparant le fond (144e') de l'ouverture (144d') de ladite empreinte en creux (144a') est plus grande que la hauteur de la paroi latérale (114) de ladite partie de fond (110).

12. Boîtier (100) selon l'une quelconque des revendications précédentes, **caractérisé en ce que** ladite partie de couvercle (120) est au moins partiellement transparente.

13. Boîtier (100) selon la revendication 12, **caractérisé en ce que** ladite partie de couvercle (120) est équipée d'une loupe (122) intégrée dans sa paroi.

14. Ensemble de protection (200), **caractérisé en ce qu'**il comporte un boîtier d'emballage et de présentation (100) selon l'une quelconque des revendications 1 à 13 et une barquette (210) délimitant un logement apte à recevoir ledit boîtier d'emballage et de présentation (100) et étant munie d'un film thermoscellable (220) refermant ledit logement.

15. Ensemble de protection (200) selon la revendication 14, **caractérisé en ce qu'**il comprend en outre un étui de protection et de conditionnement (230) apte à entourer ladite barquette (210).

16. Ensemble de protection (200) selon la revendication 15, **caractérisé en ce que** ledit étui de protection et de conditionnement (230) porte des moyens de reconnaissance visuelle (240) du type d'ensemble d'ostéosynthèse (10) qui est contenu dans ledit boîtier d'emballage et de présentation (100).

17. Ensemble de protection (200) selon l'une quelconque des revendications 14 à 16, **caractérisé en ce que** ledit étui de protection comporte (230) une partie de fond et une partie de couvercle mobiles l'un par rapport à l'autre entre une position d'ouverture et une position de fermeture, et **en ce que** ledit étui de protection comporte en outre des moyens de fermeture réversible disposés sur ladite partie de fond et sur ladite partie de couvercle qui sont aptes à coopérer dans ladite position de fermeture.

18. Coffret de présentation (300) pour la distribution d'ensembles d'ostéosynthèse (10) comprenant une partie de fond (310) équipée de compartiments (310a), au moins un compartiment 310a comprenant un ensemble de protection (200) selon la revendication 17, et au moins une partie de couvercle (320a, 320b) mobile par rapport à ladite partie de fond (310) et destinée à refermer de manière réversible au moins partiellement l'espace (330) délimité par la partie de fond (310).

## Claims

1. A packaging and display box (100) for an osteosynthesis kit (10), said osteosynthesis kit (10) comprising at least one plate (12) and at least one osteosynthesis screw (14), the box comprising a bottom portion (110) and at least one lid portion (120), said bottom portion comprising a bottom wall (112) and a side wall (114) upwardly extending the bottom wall (112) around its entire periphery, thereby defining a storage space (130), said box further comprising a heat-sealable film (150) closing said storage space (130) and means (140) for housing said osteosynthesis kit (10), **characterized in that** functional means (160) make said lid portion (120) movable relative to said bottom portion between an open position and a closed position, **in that** said box further comprises reversible closure means (170) placed on said bottom portion (110) and on said lid portion (120) and suitable for co-operating in said closed position, and **in that** said housing means (140) are suitable for being held in said storage space (130) and comprise at least one insert (140, 144, 146), said housing means being provided with at least one cavity (142) for receiving at least one osteosynthesis plate (12) and at least one cell (144a, 144a') for receiving at least one osteosynthesis screw (12).

2. A box (100) according to claim 1, **characterized in that** said cavity (142) includes a support face (142a) for supporting said osteosynthesis plate (12), which face is provided with hollow zones (142b, 142b').

3. A box (100) according to claim 2, **characterized in that** said support face (142a) is also provided with projecting zones (142c).

4. A box (100) according to claim 3, **characterized in that** said support face for supporting said osteosynthesis plate is provided with ribs (142c) and with grooves (142b').

5. A box (100) according to any one of preceding claims, **characterized in that** said cavity (142) includes a support face (142a) for supporting said osteosynthesis plate (12), which face is provided with retaining means (142b, 141) for co-operating reversibly with said osteosynthesis plate (12).

6. A box (100) according any one of the preceding claims, **characterized in that** said face of the bottom wall (112) facing away from said storage space (130) is provided with an identification medium (180) carrying information relating to said osteosynthesis kit (10), in particular as a bar code (181).

7. A box (100) according to any one of the preceding claims, **characterized in that** said housing means are formed by a one-piece insert (140).

8. A box (100) according to any one of claims 1 to 6, **characterized in that** said housing means comprise at least one first insert (140) including said cavity (142), and at least one second insert (144) including said cell (144a).

9. A box (100) according to claim 8, **characterized in that** said housing means further comprise a third insert (146) defining at least one cell (144a') for receiving an osteosynthesis screw (14).

10. A box (100) according to any one of the preceding claims, **characterized in that** said cell (144a, 144a') is formed by a hollow recess of shape that is complementary to said screw (14) so that the head of the screw (14) is situated in the opening (144d) of said recess.

11. A box (100) according to claim 10, **characterized in that** said hollow recess (144a') presents a longitudinal direction going from its opening (144d') to its bottom (144e'), said longitudinal direction being inclined relative to said bottom wall (112) so that the distance between the bottom (144e') and the opening (144d') of said hollow recess (144a') is longer than the height of the side wall (114) of said bottom portion (110).

12. A box (100) according to any one of the preceding claims, **characterized in that** said lid portion (120) is at least partially transparent.

13. A box (100) according to claim 12, **characterized in that** said lid portion (120) is provided with a magnifying glass (122) integrated in its wall.

14. A protective unit (200) comprising a packaging and display box (100) according to any one of claims 1 to 13 together with a tray (210) defining housing suitable for receiving said packaging and display box (100) and provided with a heat-sealable film (220) closing said housing.

15. A protective unit (200) according to claim 14, **characterized in that** it further comprises a protective and packaging case (230) suitable for surrounding said tray (210).

16. A protective unit (200) according to claim 15, **characterized in that** said protective and packaging tray (230) carries means (240) for visually identifying the type of osteosynthesis kit (10) contained in said packaging and display box (100).

17. A protective unit (200) according to any one of claims 14 to 16, **characterized in that** said protective case (230) comprises a bottom portion and a lid portion that are movable relative to each other between an open position and a closed position, and **in that** said protective case further comprises reversible closure means disposed on said bottom portion and on said lid portion and suitable for co-operating in said closed position.

18. A display chest (300) for distributing osteosynthesis kits (10), the display chest comprising a bottom portion (310) fitted with compartments (310a), at least one compartment (310a) comprising a protective unit (200) according to claim 17, and at least one lid portion (320a, 320b) movable relative to said bottom portion (310) and designed to close in reversible manner at least part of the space (330) defined by the bottom portion (310).

## Patentansprüche

1. Verpackungs- und Präsentationsgehäuse (100) für eine Osteosyntheseeinheit (10), wobei die Osteosyntheseeinheit (10) wenigstens eine Platte (12) und wenigstens eine Osteosyntheseschraube (14) umfaßt, wobei das Gehäuse folgendes umfaßt, nämlich einen Bodenteil (110) und wenigstens einen Deckelteil (120), wobei der Bodenteil eine bodenseitige Wand (112) und eine Seitenwand (114) aufweist, welche die bodenseitige Wand (112) über ihren gesamten Umfang nach oben fortsetzt und damit einen Ablageraum (130) begrenzt, wobei das Gehäuse ferner einen den Ablageraum (130) wieder verschließenden heißsiegelbaren Film (150) sowie Mittel (140) zur Aufnahme der Osteosyntheseeinheit (10) aufweist, **dadurch gekennzeichnet, daß** der Deckelteil (120) durch Funktionsmittel (160) gegenüber dem Bodenteil zwischen einer Öffnungsstellung und einer Schließstellung beweglich gemacht ist, daß das Gehäuse ferner Mittel (170) zum reversiblen Verschließen aufweist, die an dem Bodenteil (110) und an dem Deckelteil (120) angeordnet sind und die geeignet sind, in der Schließstellung zusammenzuwirken, und daß die Aufnahmemittel (140) geeignet sind, in dem Ablageraum (130) gehalten zu werden und wenigstens einen Einsatz (140, 144, 146) umfassen, wobei die Aufnahmemittel mit wenigstens einer Vertiefung (142) versehen sind, die dazu bestimmt ist, wenigstens eine Osteosyntheseplatte (12) aufzunehmen, sowie mit wenigstens einer Wabe (144a, 144a'), welche dazu bestimmt ist, wenigstens eine Osteosyntheseschraube (12) aufzunehmen.

2. Gehäuse (100) nach Anspruch 1, **dadurch gekennzeichnet, daß** die Vertiefung (142) eine Fläche (142a) zum Tragen der Osteosyntheseplatte (12) aufweist, die mit Hohlbereichen (142b, 142b') versehen ist.

3. Gehäuse (100) nach Anspruch 2, **dadurch gekennzeichnet, daß** die Tragfläche (142a) außerdem mit vorspringenden Bereichen (142c) versehen ist.

4. Gehäuse (100) nach Anspruch 3, **dadurch gekennzeichnet, daß** die Fläche zum Tragen der Osteosyntheseplatte mit Rippen (142c) und Nuten (142b') versehen ist.

5. Gehäuse (100) nach einem der vorhergehenden Ansprüche, **dadurch gekennzeichnet, daß** die Vertiefung (142) eine Fläche (142a) zum Tragen der Osteosyntheseplatte (12) aufweist, die mit Haltemitteln (142b, 141) versehen ist, welche dazu bestimmt sind, mit der Osteosyntheseplatte (12) reversibel zusammenzuwirken.

6. Gehäuse (100) nach einem der vorhergehenden Ansprüche, **dadurch gekennzeichnet, daß** die dem Ablageraum (130) abgewandte Seite der bodenseitigen Wand (112) mit einem ldentifikationsträger (180) versehen ist, welcher Informationen in Verbindung mit der Osteosyntheseeinheit (10), insbesondere in Form eines Barcodes (181) trägt.

7. Gehäuse (100) nach einem der vorhergehenden Ansprüche, **dadurch gekennzeichnet, daß** die Aufnahmemittel von einem einteiligen Einsatz (140) gebildet sind.

8. Gehäuse (100) nach einem der Ansprüche 1 bis 6, **dadurch gekennzeichnet, daß** die Aufnahmemittel wenigstens einen mit der Vertiefung (142) versehenen ersten Einsatz (140) sowie wenigstens einen mit der Wabe (144a) versehenen zweiten Einsatz (144) umfassen.

9. Gehäuse (100) nach Anspruch 8, **dadurch gekennzeichnet, daß** die Aufnahmemittel ferner einen dritten Einsatz (146) umfassen, der wenigstens eine für die Aufnahme einer Osteosyntheseschraube (14) bestimmte Wabe (144a') begrenzt.

10. Gehäuse (100) nach einem der vorhergehenden Ansprüche, **dadurch gekennzeichnet, daß** die Wabe (144a, 144a') von einer Hohlvertiefung gebildet ist, die eine ergänzende Form der Schraube (14) aufweist, so daß sich der Kopf der Schraube (14) in der Öffnung (144d) der Vertiefung befindet.

11. Gehäuse (100) nach Anspruch 10, **dadurch gekennzeichnet, daß** die Hohlvertiefung (144a') von ihrer Öffnung (144d') bis zu ihrem Grund (144e') eine Längsrichtung aufweist, wobei die Längsrichtung gegenüber der bodenseitigen Wand (112) geneigt ist, so daß der Abstand zwischen dem Grund (144e') und der Öffnung (144d') der Hohlvertiefung (144a') größer ist als die Höhe der Seitenwand (114) des Bodenteils (110).

12. Gehäuse (100) nach einem der vorhergehenden Ansprüche, **dadurch gekennzeichnet, daß** der Deckelteil (120) wenigstens teilweise transparent ist.

13. Gehäuse (100) nach Anspruch 12, **dadurch gekennzeichnet, daß** der Deckelteil (120) mit einer in seine Wand integrierten Lupe (122) ausgestattet ist.

14. Schutzeinheit (200), **dadurch gekennzeichnet, daß** sie ein Verpackungs- und Präsentationsgehäuse (100) nach einem der Ansprüche 1 bis 13 sowie einen Behälter (210) umfaßt, der eine Aufnahme begrenzt, welche geeignet ist, das Verpackungs-und Präsentationsgehäuse (100) aufzunehmen, und der mit einem die Aufnahme wieder verschließenden heißsiegelbaren Film (220) versehen ist.

15. Schutzeinheit (200) nach Anspruch 14, **dadurch gekennzeichnet, daß** sie ferner ein Schutz- und Verpackungsgehäuse (230) umfaßt, welches geeignet ist, den Behälter (210) zum umschließen.

16. Schutzeinheit (200) nach Anspruch 15, **dadurch gekennzeichnet, daß** das Schutz- und Verpackungsgehäuse (230) Mittel zur visuellen Erkennung (240) der Art der in dem Verpackungs- und Präsentationsgehäuse (100) enthaltenen Osteosyntheseeinheit (10) trägt.

17. Schutzeinheit (200) nach einem der Ansprüche 14 bis 16, **dadurch gekennzeichnet, daß** das Schutzgehäuse (230) einen Bodenteil und einen Deckelteil aufweist, die zwischen einer Öffnungsstellung und einer Schließstellung zueinander beweglich sind, und daß das Schutzgehäuse ferner Mittel zum reversiblen Verschließen aufweist, die an dem Bodenteil und an dem Deckelteil angeordnet und geeignet sind, in der Schließstellung zusammenzuwirken.

18. Präsentationskoffer (300) für den Vertrieb von Osteosyntheseeinheiten (10), mit einem mit Fächern (310a) ausgestatteten Bodenteil (310), wobei wenigstens ein Fach (310a) eine Schutzeinheit (200) nach Anspruch 17 umfaßt, sowie mit wenigstens einem Deckelteil (320a, 320b), der gegenüber dem Bodenteil (310) beweglich und dazu bestimmt ist, den durch den Bodenteil (310) begrenzten Raum (330) wenigstens teilweise reversibel zu verschließen.
